# EUROPEAN PATENT SPECIFICATION

(11) **EP 1 945 785 B1**
(45) Date of publication and mention of the grant of the patent: **10.02.2010**
(21) Application number: 06807113.3
(22) Date of filing: 10.10.2006
(51) Int. Cl.: C12P 7/04

(54) **PROCESS FOR PREPARING 1,1,1-TRIFLUOROISOPROPANOL PREDOMINANTLY COMPRISING ONE ENANTIOMER**
VERFAHREN ZUR HERSTELLUNG VON VORWIEGEND EIN ENANTIOMER ENTHALTENDES 1,1,1-TRIFLUORISOPROPANOL
PROCÉDÉ DE SYNTHÈSE DE 1,1,1-TRIFLUOROISOPROPANOL PRINCIPALEMENT CONSTITUÉ D'UN ÉNANTIOMÈRE

(30) Priority: 11.11.2005 DE 102005054282
(43) Date of publication of application: 23.07.2008
(73) Proprietor: Evonik Degussa GmbH, 45128 Essen (DE)
(72) Inventor: DODERER, Kai, 63110 Rodgau (DE); GRÖGER, Harald, 91056 Erlangen (DE); MAY, Oliver, 52076 Aachen (DE)
(86) International application number: PCT/EP2006/067228
(87) International publication number: WO 2007/054411

(56) References cited:
- NAKAMURA ET AL: "Different stereochemistry for the reduction of trifluoromethyl ketones and methyl ketones catalyzed by alcohol dehydrogenase from Geotrichum" TETRAHEDRON LETTERS, vol. 37, 1996, pages 5727-5730, XP004030523
- NAKAMURA ET AL: "Asymmetric reduction of trifluoromethyl ketones containing a sulfur functionality by the alcohol dehydrogenase from Geotrichum" TETRAHEDRON, vol. 54, 1998, pages 8393-8402, XP004124021
- INOUE ET AL: "Purification and characterization of a novel alcohol dehydrogenase from Leifsonia sp. strain S749: a promising biocatalyst for an asymmetric hydrogen transfer bioreduction" APPLIED AND ENVIRONMENTAL MICROBIOLOGY, vol. 71, July 2005 (2005-07), pages 3633-3641, XP002405894
- BUCCIARELLI ET AL: "Asymmetric reduction of trifluoromethyl and methyl ketones by yeast; An improved method" SYNTHESIS, 1983, pages 897-899, XP002398751 cited in the application
- HAMADA ET AL: "Efficient synthesis of optically pure 1,1,1,-trifluoro-2-alkanols through lipase-catalyzed acylation in organic media" JOURNAL OF ORGANIC CHEMISTRY, vol. 61, 1996, pages 2332-2336, XP002405897
- BOTT ET AL: "Steric effects. A study of a rationally designed system" JOURNAL OF THE AMERICAN CHEMICAL SOCIETY, vol. 102, 1980, pages 5618-5626,

## Description

The invention relates to a process for preparing 1,1,1-trifluoroisopropanol in which a particular enantiomer predominates, by enantioselective enzymatic reduction of 1,1,1-trifluoroacetone.

Only a few methods are known for synthesizing 1,1,1-trifluoroisopropanol (alternative name: 1,1,1-trifluoro-2-hydroxypropane) which comprises predominantly a particular enantiomer as building block of pharmaceutical interest. Thus, for example, the enantiomers can be selected by a separation, following the preparation, of the racemate formed, as described by Yonezawa et al. (T. Yonezawa, Y. Sakamoto, K. Nogawa, T. Yamazaki, T. Kitazume, Chem. Lett. 1996, 855-856). However, racemate resolutions of this type are restricted to a maximum yield of 50% of the overall product previously prepared.

Without doubt the most efficient synthetic route in principle is direct asymmetric conversion of the corresponding ketone "1,1,1-trifluoroacetone" into the corresponding alcohol, with the desired enantiomer being obtained directly in this case. This is possible in principle with use of a chiral reducing agent or, alternatively, of a chiral catalyst and of a reducing agent. The latter method is moreover preferred for reasons of efficiency and because of the distinctly improved atom economy. However, to date only two studies on the preparation of 1,1,1-trifluoroisopropanol predominantly comprising one enantiomer (also described as "enantiomer-enriched" hereinafter) with the aid of an enantioselective reduction of 1,1,1-trifluoroacetone have been disclosed, and are described below. In view of the large number of methods developed for asymmetric reduction of ketones, this is extremely surprising. Thus, Brown et al. obtained on use of a chiral reducing agent for the stereoselective reduction of 1,1,1-trifluoroacetone the corresponding alcohol with an enantiomeric excess of 89% ee (P.V. Ramachandran, A.V. Teodorovic, H.C. Brown, Tetrahedron 1993, 49, 1725-1738). The disadvantage from the industrial viewpoint in this case is, besides the enantiomeric excess being below 90% ee, the use of the chiral reducing agent "(-)-B-chlorodiisopinocampheylborane", which is required in stoichiometric amounts. Moreover, stoichiometric amounts of a camphor derivative are required as chiral auxiliary to prepare this organoboron compound.

An alternative synthesis of enantiomer-enriched 1,1,1-trifluoroisopropanol is based on a direct enzymatic reduction of 1,1,1-trifluoroacetone with use of alcohol dehydrogenases. Alcohol dehydrogenases belong to the enzyme class EC 1 and catalyse the (reversible) reaction of a carbonyl compound to form an alcohol functionality. The Prelog rule applies to the selectivity in this case, according to which a conversion preferentially to the corresponding (*R*) or (*S*) enantiomer takes place depending on the respective size of the radicals adjacent to the carbonyl group (K. Faber, Biotransformations in Organic Chemistry, 4th edition, Springer, Berlin, 2000, Chapter 2.1.1, pp. 166-167). Consequently, reduction of ketones with radicals of similar size adjacent to the carbonyl group, as is also the case in 1,1,1-trifluoroacetone for example, is problematic. Accordingly, no highly selective reductions with enzymes have been disclosed to date for 1,1,1-trifluoroisopropanol. Thus, the use of baker's yeast reported by Bucciarelli et al. for the reduction of 1,1,1-trifluoroacetone to form (*S*)-1,1,1-trifluoroisopropanol leads to an enantiomeric excess of only 80.3% ee [M. Bucciarelli, A. Forni, I. Moretti, G. Torre, Synthesis 1983, 897-899] and is thus significantly below the enantiomeric excess which is desirable for industrial applications, >90% ee, in particular >95% see and very preferably >99% ee. For example, the requirement for pharmaceutical applications is that the optically active products have an enantiomeric excess of in each case >99% ee (concerning the definition of "ee" and further explanations, see, for example: K. Faber, Biotransformations in Organic Chemistry, 4th edition, Springer, Berlin, 2000, Chapter 2.1.1, pp. 28-52). In contrast thereto, numerous examples are known of for example alcohol dehydrogenase-catalysed reductions of ketones which have sterically distinctly different substituents adjacent to the carbonyl group, and proceed with enantioselectivities of >90% ee, in particular >95% ee and very preferably >99% ee [see, inter alia: K. Nakamura, T. Matsuda in: Enzyme Catalysis in Organic Synthesis (editors: K. Drauz, H. Waldmann), Volume III, Wiley-VCH, 2nd edition, 2002, pp. 991-1047]. See also Nakamura et. al., Tetrahedron Letters, 1996, 5727-5730.

The object of the present invention was to indicate a process for preparing 1,1,1-trifluoroisopropanol predominantly comprising one enantiomer which makes it possible to prepare these compounds with a high enantiomeric excess of >90% ee, in particular >95% ee and very preferably >99% ee.

This object is achieved by a process according to Claim 1. Preferred embodiments are represented in the dependent claims. The process makes use of the enantioselective reduction of 1,1,1-trifluoroacetone.

Surprisingly, enantiomeric excesses of up to >99% ee are obtained on use of the process of the invention, unexpectedly in the light of the state of the art hitherto. Thus, in the light of the experimental results published hitherto, ee values of distinctly <90% ee, as well as, on the basis of the Prelog rule - as a result of the similarity of the two radicals CH₃ and CF₃ adjacent to the carbonyl group - in principle a lower selectivity was to be expected. By contrast, however, excellent enantioselectivities of >90% ee, in particular >95% and very particularly preferably >99% ee are obtained in the process of the invention.

Alcohol dehydrogenases which can be employed in the process of the invention are in principle all enzymes of this type which are known to the skilled person as long as they are able to catalyse the conversion/reaction used in the process of the invention. This can be found out by routine experiments.

An alcohol dehydrogenase which is preferably employed is one which catalyses a stereoselective conversion even if the radicals adjacent to the carbonyl group have a similar steric size.

It is particularly preferred to employ at least one alcohol dehydrogenase from the organisms *Rhodococcus erythropolis* (S-ADH), *Arthrobacter paraffineus* (S-ADH) or *Lactobacillus kefir* (R-ADH) (ADH from *R. erythropolis*: a) EP 1499716; b) K. Abokitse, W. Hummel, Cloning, sequence analysis, and heterologous expression of the gene encoding a (*S*)-specific alcohol dehydrogenase from *Rhodococcus erythropolis* DSM 43297, Appl. Microbiol. Biotechnol. 2003, 62, 380-386; c) PCT/EP2005/06215). (ADH from *A. paraffineus*: WO 2005103239) (ADH from *Lactobacillus kefir*: a) EP 456107; b) C.W. Bradshaw, W. Hummel, C.-H. Wong, Lactobacillus kefir Alcohol Dehydrogenase: A Useful Catalyst for Synthesis, J. Org. Chem. 1992, 57, 1532-1536; c) PCT/EP2005/06215).

In a further preferred embodiment, the invention also relates to a process of the invention in which at least one alcohol dehydrogenase originates from an organism selected from the group consisting of *Lactobacillus kefir, Rhodotorula glutinis, Carnobacterium divergens, Streptococcus ferus, Blastobacter natatorius, Sporidiobolus salmonicolor, Pichia haplophila, Pichia pastoris, Kluyveromyces marxianus, Pichia carsonii, Saccharomyces cerevisiae* and *Rhodococcus erythropolis.*

A bacterial alcohol dehydrogenase is preferably employed.

It is possible in principle for the alcohol dehydrogenase(s) to be employed in the forms familiar to the skilled person (see below) in the process of the invention. Since, however, alcohol dehydrogenases as oxidoreductases are cofactor-dependent enzymes, the cofactor necessary for the enzyme employed must be present in sufficient quantity in the reaction mixture to carry out the reduction successfully in order to be able to ensure complete transformation of the ketone. Since these cofactors are relatively costly molecules, the use of minimum quantities of cofactor is a crucial advantage for economic reasons. One possibility for being able to employ the cofactor below the stoichiometrically necessary quantity is to regenerate it by a second biocatalyst which is present in the mixture. A system of this type operates in such a way that an enzymatic conversion of a (e.g. organic) compound proceeds with "consumption" of a cofactor, and this cofactor is regenerated *in situ* by a second enzymatic system. The result thereof is thus a reduction in the quantity to be employed of costly cofactors.

Reaction by means of a coupled enzymatic system thus represents an advantageous procedure.

Such coupled systems are mentioned for example in DE-A 10233046 or DE-A 10233107.

The enzyme which regenerates the employed cofactor is dependent firstly on the employed cofactor but secondly also on the cosubstrate to be oxidized or reduced. Some enzymes for regenerating NAD(P)H are mentioned in Enzyme Catalysis in Organic Synthesis, Ed.: K. Drauz, H. Waldmann, 1995, Vol. I, VCH, page 721. Of commercial interest and also obtainable on a large scale, and currently employed for synthesizing amino acids, for these reasons the so-called formate dehydrogenase (FDH) (see also DE-A 10233046) and alternatively the so-called glucose dehydrogenase is advantageously used. They can therefore also be used preferably in the process of the invention for regenerating the cofactor. The FDH very particularly preferably originates from the organism *Candida boidinii.* It is also possible to employ further developed mutants thereof, e.g. those described in DE-A 19753350. It is further possible and preferred to employ a glucose dehydrogenase from *Bacillus subtilis.* However, substrate-coupled regeneration is also possible, for example through use of iso-propanol (examples of the methodology for cofactor regeneration with iso-propanol: a) W. Stampfer, B. Kosjek, C. Moitzi, W. Kroutil, K. Faber, Angew. Chem. 2002, 114, 1056-1059; b) M. Wolberg, W. Hummel, C. Wandrey, M. Müller, Angew. Chem. 2000, 112, 4476-4478).

It is possible in the process of the invention for the stereospecific conversion/reaction to take place in any media suitable for this reaction. The catalysis can be carried out for example in purely aqueous solutions or in hydrous media supplemented with organic solvents. Possibilities in this connection are single phase or multiphase systems. The chosen reaction medium is not limiting for the process of the invention as long as the chosen enzyme is able to catalyse the desired stereoselective reaction therein with the yield described herein.

It is advantageous for the process - with a view to a high volumetric productivity - to be carried out with high initial substrate concentrations. These are typically >50 g/l, preferably >100 g/l and very preferably >150 g/l. The substrate concentrations can moreover optionally be maintained by continuously feeding in fresh substrate solution during the catalytic conversion, especially when a whole-cell catalyst described below is employed.

The process can in principle be carried out at any suitable temperature. The aim for the skilled person in this connection is preferably to obtain a maximum yield of the desired product in maximum purity and in minimum time. In addition, the employed enzymes should be sufficiently stable at the temperatures employed and the reaction should proceed with maximum enantioselectivity. On use of enzymes from thermophilic organisms it is perfectly possible for example to reach temperatures of 100°C. The primary aim is for the temperature to be at the catalytic optimum of the enzyme employed. A lower limit which is certainly sensible in aqueous systems is -15°C. A temperature range between 10°C and 60°C, particularly preferably between 20°C and 40°C, is preferred for the process of the invention and is primarily determined in accordance with the abovementioned criteria.

The pH during the reaction is likewise primarily determined in accordance with the stabilities of the employed enzymes and cofactors and can be ascertained by determining the conversion rates and be set correspondingly for the process of the invention. A range preferred for enzymes will generally be from pH 5 to 11, but this may also in exceptional cases be higher or lower if one of the employed enzymes has its catalytic maximum at a lower or higher value. A pH range in which the reaction is carried out in the process of the invention can preferably be from 5.5 to 10.0, in particular from 6.0 to 9.0.

Although the process described herein can also be carried out with isolated enzymes in suitable reaction media, the process of the invention is carried out in a particularly preferred embodiment by employing a whole-cell catalyst, that is a system comprising (at least one) whole-cell(s) for the reaction, the cells preferably being able to express simultaneously the desired alcohol dehydrogenase and the cofactor-regenerating enzyme (for the design of processes using whole-cell catalysts for enantioselective reduction, see, for example, inter alia: PCT/EP2005/06215).
The cell(s) thus express(es) preferably at least one enzyme (polypeptide) having an alcohol dehydrogenase activity and at least one having an activity for regenerating the cofactor employed. These enzymes and/or the cells employed preferably originate from the organisms mentioned hereinbefore.

It is possible alternatively - on use of cofactor regeneration with iso-propanol - also to use cells which preferably express at least one enzyme (polypeptide) having an alcohol dehydrogenase activity and only optionally one having an activity for regenerating the cofactor employed.

Suitable microorganisms which can in principle be cited are all the organisms known to the skilled person for this purpose, such as, for example, yeasts such as *Hansenula polymorpha, Pichia sp., Saccharomyces cerevisiae,* prokaryotes such as *E. coli, Bacillus subtilis* or eukaryotes such as mammalian cells, insect cells, etc. It is possible and preferred to employ *E. coli* strains for this purpose. Very particular preference is given to: *E. coli* XL1 Blue, NM 522, JM101, JM109, JM105, RR1, DH5α, TOP 10⁻ or HB101. These strains are generally known and can be purchased.

An organism like that mentioned in DE-A 10155928 is preferably employed as host organism. The advantage of such an organism is the simultaneous expression of both the polypeptide systems suitable for the process of the invention, so that only one recombinant (genetically modified) organism need be employed for the process of the invention.

In order to adjust the expression of the polypeptides (enzymes) in relation to the desired catalytic activity, it is possible for the corresponding encoding nucleic acid sequences to be present on different plasmids with different copy numbers and/or for promoters of different strengths to be used for different strengths of expression of the nucleic acid sequences. With such adjusted enzyme systems there is advantageously no accumulation of an intermediate compound, and the reaction under consideration can proceed at an optimal overall rate. This is, however, sufficiently well known to the skilled person (Gellissen, G.; Piontek, M.; Dahlems, U.; Jenzelewski, V.; Gavagan, J.W.; DiCosimo, R.; Anton, D.L.; Janowicz, Z.A. (1996), Recombinant Hansenula polymorpha as a biocatalyst. Coexpression of the spinach glycolate oxidase (GO) and the S. cerevisiae catalase T (CTT1) gene, Appl. Microbiol. Biotechnol. 46, 46-54; Farwick, M.; London, M.; Dohmen, J.; Dahlems, U.; Gellissen, G.; Strasser, A.W.; DE-A 19920712). Production of the microorganism which is used as "whole-cell catalyst" and is genetically modified where appropriate can in principle take place by methods known to the skilled person (Sambrook, J.; Fritsch, E.F. and Maniatis, T. (1989), Molecular cloning: a laboratory manual, 2nd ed., Cold Spring Harbor Laboratory Press, New York; Balbas, P. and Bolivar, F. (1990), Design and construction of expression plasmid vectors in E. coli, Methods Enzymol. 185, 14-37; Rodriguez, R.L. and Denhardt, D.T. (eds) (1988), Vectors: a survey of molecular cloning vectors and their uses, 205-225, Butterworth, Stoneham). Concerning the techniques used for the general procedure (PCR, cloning, expression, etc.), reference may be made to the following literature and that cited therein: Universal GenomeWalker™ Kit User Manual, Clontech, 3/2000 and literature cited therein; Triglia T.; Peterson, M.G. and Kemp, D.J. (1988), A procedure for in vitro amplification of DNA segments that lie outside the boundaries of known sequences, Nucleic Acids Res. 16, 8186; Sambrook, J.; Fritsch, E.F. and Maniatis, T. (1989), Molecular cloning: a laboratory manual, 2nd ed., Cold Spring Harbor Laboratory Press, New York; Rodriguez, R.L. and Denhardt, D.T. (eds) (1988), Vectors: a survey of molecular cloning vectors and their uses, Butterworth, Stoneham.

The reaction system is preferably employed for example in a stirred vessel, a cascade of stirred vessels or in membrane reactors, which can be operated both in batch operation and continuously. However, any type of system in which the process of the invention can be carried out is suitable.

In the context of the invention, a membrane reactor means any reaction vessel in which the catalyst is enclosed in a reactor, while low molecular weight substances can be fed into the reactor or are able to leave it. It is moreover possible for the membrane to be integrated directly in the reaction chamber or to be incorporated in a separate filtration module outside, with the reaction solution flowing continuously or intermittently through the filtration module, and the retentate being returned to the reactor. Suitable embodiments are described inter alia in WO98/22415 and in Wandrey et al. in Jahrbuch 1998, Verfahrenstechnik und Chemieingenieurwesen, VDI, pp. 151 et seq.; Wandrey et al. in Applied Homogeneous Catalysis with Organometallic Compounds, Vol. 2, VCH 1996, pp. 832 et seq.; Kragl et al., Angew. Chem. 1996, 6, 684 et seq. The continuous procedure, which is possible in this apparatus besides the batch and semicontinuous procedure, can moreover be carried out for example in cross-flow filtration mode or as dead-end filtration. Both process variants are described in principle in the prior art (Engineering Processes for Bioseparations, Ed.: L.R. Weatherley, Heinemann, 1994, 135-165; Wandrey et al., Tetrahedron Asymmetry 1999, 10, 923-928).

For use, the polypeptides under consideration for the process of the invention can be used either in free form as homogeneously purified compounds or as recombinantly produced enzyme. It is furthermore possible for these polypeptides also to be employed as constituent of an intact "guest organism" (genetically modified microorganism) or in conjunction with a mass of host organism cells which have if required been purified and, where appropriate, disrupted.

It is likewise possible to use the enzymes in immobilized form (Sharma B.P.; Bailey L.F. and Messing R.A. (1982), Immobilisierte Biomaterialiern - Techniken und Anwendungen, Angew. Chem. 94, 836-852). The immobilization is preferably effected by lyophilization (Paradkar, V.M.; Dordick, J.S. (1994), Aqueous-Like Activity of α-Chymotrypsin Dissolved in Nearly Anhydrous Organic Solvents, J. Am. Chem. Soc. 116, 5009-5010; Mori, T.; Okahata, Y. (1997), A variety of lipi-coated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solubilize the enzyme in active form in toluene, Biocatalysis 6, 291-305). Very particular preference is given to lyophilization in the presence of surface-active substances such as, for example, Aerosol OT, polyvinylpyrrolidone, polyethylene glycol (PEG) or Brij 52 (diethylene glycol monocetyl ether) (Kamiya, N.; Okazaki, S.-Y.; Goto, M. (1997), Surfactant-horseradish peroxidase complex catalytically active in anhydrous benzene, Biotechnol. Tech. 11, 375-378), without being restricted thereto. Particular preference is given to immobilization on Eupergit^{®}, in particular Eupergit C^{®} and Eupergit 250L^{®} (Röhm) (Eupergit.RTM. C, a carrier for immobilization of enzymes of industrial potential. Katchalski-Katzir, E.; Kraemer, D. M. Journal of Molecular Catalysis B: Enzymatic (2000), 10(1-3), 157-176).

Preference is likewise given to immobilization on Ni-NTA in combination with a polypeptide supplemented with a His tag (hexa-histidine) (Purification of proteins using polyhistidine affinity tags. Bornhorst, Joshua A.; Falke, Joseph J. Methods in Enzymology (2000), 326, 245-254).

Use as CLECs is likewise conceivable (St. Clair, N.; Wang, Y.-F.; Margolin, A.L. (2000), Cofactor-bound cross-linked enzyme crystals (CLEC) of alcohol dehydrogenase, Angew. Chem. Int. Ed. 39, 380-383).

These measures are also suitable for generating polypeptides which show catalytic activity in mixtures of aqueous and organic solvents, or in a completely organic medium, from those which are destabilized by organic solvents.

The enantiomer-enriched 1,1,1-trifluoroisopropanol is prepared by the process of the invention by preferably initially dissolving the ketone corresponding to the desired final product (1,1,1-trifluoroacetone) in a preferably hydrous solvent, optionally adding to this solution all the additives which are necessary for the biocatalyst and stabilize it, and adjusting the pH where appropriate, adding the biocatalyst to the solution, and thus carrying out the reduction of the ketone to form the desired (*R*)- or (*S*)-enantiomer-enriched 1,1,1-trifluoroisopropanol.

In a particularly preferred embodiment, the 1,1,1-trifluoroacetone is directly dissolved in a cell medium which is suitable for the biocatalyst (expressing the desired enzymes), the biocatalyst and optionally the cofactors necessary for the enzymes are added, and the catalytic conversion to the desired enantiomer is carried out at a temperature at which the biocatalyst is stable and the enzymes show a high activity for the respective reaction catalysed thereby.

Alternatively, the sequence of addition of the respective components can, however, also be varied as desired. Thus, a further preferred embodiment consists of adding the biocatalyst before the addition of the 1,1,1-trifluoroacetone.

"Enantiomer-enriched" or "enantiomer enriched" or "predominantly comprising one enantiomer" refers to the presence of >50% of one enantiomer in relation to its optical antipode in the mixture.

The structures of 1,1,1-trifluoroacetone, (*S*)-1,1,1-trifluoroisopropanol and (*R*)-1,1,1-trifluoroisopropanol are depicted graphically below:

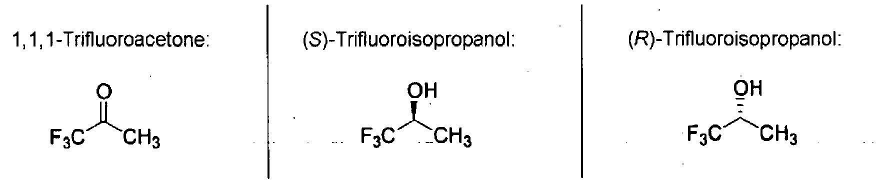

### Experimental examples:

### Example 1:

A reaction solution (2 ml) consisting of 1,1,1-trifluoroacetone (10 mM substrate concentration), cofactor (10 mM cofactor concentration; NADPH or NADH depending on the cofactor preference of the enzyme used) and enzyme solution of the particular alcohol dehydrogenase (prepared from a standard cell disruption by bead mill of 200 mg of wet biomass (*E. coli,* DSM 14459) comprising the alcohol dehydrogenase in overexpressed form in 0.8 ml of buffer solution) is stirred at a reaction temperature of 30°C for 24 hours. After 24 hours, the reaction mixture is examined for the formation of the desired product (*S*)- or (*R*)-1,1,1-trifluoroisopropanol. The results are shown in the table below.

**Table 1: Enantioselective reduction of 1,1,1-trifluoroacetone**

| Experiment No. | Enzyme^{a)} | Reaction time [h] | Conversion [%] | ee [%] ^{b)} |
|---|---|---|---|---|
| 1 | RE-ADH | 24 | 94 | >99 (*S*) |
| 2 | AP-ADH | 24 | 4 | >99 (*S*) |
| 3 | LK-ADH | 24 | 80 | 90 (*R*) |

| | | | | |
|---|---|---|---|---|
| a) RE-ADH = alcohol dehydrogenase from *Rhodococcus erythropolis,* concerning this, see inter alia: PCT/EP2005/06215; AP-ADH = alcohol dehydrogenase from *Arthrobacter paraffineus,* concerning this, see inter alia: WO 2005103239: LK-ADH = alcohol dehydrogenase from *Lactobacillus kefir,* concerning this, see inter alia: PCT/EP2005/06215; b) the enantiomer preferentially formed is indicated in parentheses in each case. | | | | |

### Example 2:

Preparative biocatalytic reduction of 1,1,1-trifluoroacetone to form (*S*)-1,1,1-trifluoroisopropanol at a substrate concentration of 100 mM by the whole-cell process (for the whole-cell catalyst and the principle of the process, see also PCT/EP/2005/06215): a reaction mixture (total volume: 50 ml) consisting of phosphate buffer (adjusted to pH 6.5), (*S*)-enantioselective whole-cell catalyst of the *E.coli* DSM14459 type (comprising an (*S*)-alcohol dehydrogenase from *R. erythropolis,* and a glucose dehydrogenase from *Bacillus subtilis,* prepared as described in the patent application PCT/EP2005/06215; amount: 1.5 g, equivalent to a cell concentration of ∼30 g of wet biomass/l), D-glucose (amount: 1.486 g, corresponding to 1.5 equivalents based on the molar amount of ketone employed) and trifluoroacetone (amount: 560.3 mg, 5 mmol, equivalent to a substrate concentration of 100 mM) is prepared in a Titrino reaction vessel. The reaction mixture is then stirred at room temperature for a reaction time of 24 hours, the pH being kept constant at ∼6.5 by adding sodium hydroxide solution (1M NaOH). The biomass is then removed by centrifugation and the conversion is determined by ¹⁹F-NMR spectroscopy on the resulting filtrate. A conversion of >95% is found in this case.

## Claims

1. Process for preparing 1,1,1-trifluoroisopropanol predominantly comprising one enantiomer by enantioselective reduction of 1,1,1-trifluoroacetone with the aid of an alcohol dehydrogenase, **characterized in that** the desired enantiomer of 1,1,1-trifluoroisopropanol is formed with an enantiomeric excess of >90% ee.

2. Process according to Claim 1,
**characterized in that** the desired enantiomer of 1,1,1-trifluoroisopropanol is formed with an enantiomeric excess of >95% ee.

3. Process according to either of the preceding claims,
**characterized in that**
the alcohol dehydrogenase employed in the process originates from an organism selected from the group consisting of *Lactobacillus kefir, Rhodotorula glutinis, Carnobacterium divergens, Streptococcus ferus, Blastobacter natatorius, Sporidiobolus salmonicolor, Pichia haplophila, Pichia pastoris, Kluyveromyces marxianus, Pichia carsonii, Saccharomyces cerevisiae* and *Rhodococcus erythropolis.*

4. Process according to Claim 1 or 2,
**characterized in that**
a bacterial alcohol dehydrogenase is employed.

5. Process according to Claim 3 or 4,
**characterized in that**
a bacterial alcohol dehydrogenase from *Rhodococcus erythropolis* is employed.

6. Process according to any of the preceding claims,
**characterized in that**
the conversion of the ketone takes place with the aid of a coupled enzymatic system.

7. Process according to Claim 6, where the coupled enzymatic system consists of an alcohol dehydrogenase and of an enzyme regenerating the cofactor of the alcohol dehydrogenase.

8. Process according to any of the preceding claims,
**characterized in that**
the initial substrate concentrations present for the conversion of the ketone are >50 g/l.

9. Process according to any of the preceding claims,
**characterized in that**
the conversion takes place in a temperature range from -15 to 100°C.

10. Process according to any of the preceding claims,
**characterized in that**
the conversion takes place at a pH of from 5 to 11.

11. Process according to any of the preceding claims,
**characterized in that**
at least one microorganism which is capable of simultaneous expression of the alcohol dehydrogenase and of an enzyme regenerating a cofactor is employed in the process.

## Patentansprüche

1. Verfahren zur Herstellung von überwiegend ein Enantiomer enthaltendem 1,1,1-Trifluorisopropanol durch enantioselektive Reduktion von 1,1,1-Trifluoraceton mit Hilfe einer Alkoholdehydrogenase, **dadurch gekennzeichnet, dass** das gewünschte Enantiomer des 1,1,1-Trifluorisopropanols mit einem Enantiomerenüberschuss von >90% ee gebildet wird.

2. Verfahren nach Anspruch 1, **dadurch gekennzeichnet, dass** das gewünschte Enantiomer des 1,1,1-Trifluorisopropanols mit einem Enantiomerenüberschuss von >95% ee gebildet wird.

3. Verfahren nach einem der vorhergehenden Ansprüche, **dadurch gekennzeichnet, dass** die in das Verfahren eingesetzte Alkoholdehydrogenase aus einem Organismus ausgewählt aus der Gruppe bestehend aus Lactobacillus kefir, Rhodotorula glutinis, Carnobacterium divergens, Streptococcus ferns, Blastobacter natatorius, Sporidiobolus salmonicolor, Pichia haplophila, Pichia pastoris, Kluyveromyces marxianus, Pichia carsonii, Saccharomyces cerevisiae und Rhodococcus erythropolis stammt.

4. Verfahren nach Anspruch 1 oder 2, **dadurch gekennzeichnet, dass** eine bakterielle Alkoholdehydrogenase eingesetzt wird.

5. Verfahren nach Anspruch 3 oder 4, **dadurch gekennzeichnet, dass** eine bakterielle Alkoholdehydrogenase aus Rhodococcus erythropolis eingesetzt wird.

6. Verfahren nach einem der vorhergehenden Ansprüche, **dadurch gekennzeichnet, dass** die Umsetzung des Ketons mit Hilfe eines gekoppelten enzymatischen Systems erfolgt.

7. Verfahren nach Anspruch 6, wobei das gekoppelte enzymatische System aus einer Alkoholdehydrogenase und einem den Cofaktor der Alkoholdehydrogenase regenerierenden Enzym besteht.

8. Verfahren nach einem der vorhergehenden Ansprüche, **dadurch gekennzeichnet, dass** die für die Umsetzung des Ketons vorliegenden Substratausgangskonzentrationen >50 g/L betragen.

9. Verfahren nach einem der vorhergehenden Ansprüche, **dadurch gekennzeichnet, dass** die Umsetzung in einem Temperaturbereich von -15 bis 100°C erfolgt.

10. Verfahren nach einem der vorhergehenden Ansprüche, **dadurch gekennzeichnet, dass** die Umsetzung bei einem pH-Wert von 5 bis 11 erfolgt.

11. Verfahren nach einem der vorhergehenden Ansprüche, **dadurch gekennzeichnet, dass** in das Verfahren wenigstens ein Mikroorganismus eingesetzt wird, der zur gleichzeitigen Expression der Alkoholdehydrogenase und eines einen Cofaktor regenerierenden Enzyms in der Lage ist.

## Revendications

1. Procédé pour préparer du 1,1,1-trifluoroisopropanol comprenant principalement un énantiomère par réduction énantiosélective de 1,1,1-trifluoroacétone à l'aide d'une alcool déshydrogénase, **caractérisé en ce que** l'énantiomère souhaité du 1,1,1-trifluoroisopropanol est formé avec un excès énantiomérique > 90 % d'ee.

2. Procédé selon la revendication 1, **caractérisé en ce que** l'énantiomère souhaité du 1,1,1-trifluoroisopropanol est formé avec un excès énantiomérique > 95 % d'ee.

3. Procédé selon l'une ou l'autre des revendications précédentes, **caractérisé en ce que** l'alcool déshydrogénase employée dans le procédé provient d'un organisme choisi dans le groupe constitué par *Lactobacillus kefir, Rhodotorula glutinis, Carnobacterium divergens, Streptococcus ferus, Blastobacter natatorius, Sporidiobolus salmonicolor, Pichia haplophila, Pichia pastoris, Kluyveromyces marxianus, Pichia carsonii, Saccharomyces cerevisiae* et *Rhodococcus erythropolis.*

4. Procédé selon la revendication 1 ou 2, **caractérisé en ce qu'**une alcool déshydrogénase bactérienne est employée.

5. Procédé selon la revendication 3 ou 4, **caractérisé en ce qu'**une alcool déshydrogénase bactérienne provenant de *Rhodococcus erythropolis* est employée.

6. Procédé selon l'une quelconque des revendications précédentes, **caractérisé en ce que** la conversion de la cétone a lieu à l'aide d'un système enzymatique couplé.

7. Procédé selon la revendication 6, dans lequel le système enzymatique couplé est constitué d'une alcool déshydrogénase et d'une enzyme régénérant le cofacteur de l'alcool déshydrogénase.

8. Procédé selon l'une quelconque des revendications précédentes, **caractérisé en ce que** les concentrations en substrat initiales présentes pour la conversion de la cétone sont > 50 g/l.

9. Procédé selon l'une quelconque des revendications précédentes, **caractérisé en ce que** la conversion à lieu dans une plage de température de -15 à 100 °C.

10. Procédé selon l'une quelconque des revendications précédentes, **caractérisé en ce que** la conversion a lieu à un pH de 5 à 11.

11. Procédé selon l'une quelconque des revendications précédentes, **caractérisé en ce qu'**au moins un microorganisme qui est capable de l'expression simultanée de l'alcool déshydrogénase et d'une enzyme régénérant un cofacteur est employé dans le procédé.
